(19) **Europäisches Patentamt**

**European Patent Office**

**Office européen des brevets**

(11) **EP 0 571 939 B1**

(12) **EUROPÄISCHE PATENTSCHRIFT**

(45) Veröffentlichungstag und Bekanntmachung des Hinweises auf die Patenterteilung:
**09.09.1998 Patentblatt 1998/37**

(51) Int Cl.⁶: **C12Q 1/34**, G01N 33/58

(21) Anmeldenummer: **93108403.2**

(22) Anmeldetag: **25.05.1993**

(54) **Mittel zur Bestimmung eines Analyts**

Means for the determination of an analyte

Moyen pour la détermination d'une analyte

(84) Benannte Vertragsstaaten:
**AT BE CH DE DK ES FR GB IT LI NL SE**

(30) Priorität: **27.05.1992 DE 4217474**

(43) Veröffentlichungstag der Anmeldung:
**01.12.1993 Patentblatt 1993/48**

(73) Patentinhaber: **BOEHRINGER MANNHEIM GMBH**
**68298 Mannheim (DE)**

(72) Erfinder:
- **Pollmann, Klaus, Dr.**
  **W-6823 Neulussheim (DE)**
- **Freitag, Helmut, Dr.**
  **W-6940 Weinheim (DE)**
- **Rothe, Anselm, Dr.**
  **W-6943 Birkenau (DE)**

(56) Entgegenhaltungen:
EP-A- 0 244 932      EP-A- 0 347 839
EP-A- 0 373 908      EP-A- 0 518 557
WO-A-90/00252        US-A- 4 446 232

**Beschreibung**

Gegenstand der Erfindung sind ein Mittel zur Bestimmung der Konzentration von Substanzen mit Hydrolaseaktivität in einer Probe sowie ein Testmittel zur Bestimmung eines Analyts in Köperflüssigkeit unter Verwendung eines Konjugats aus einer Hydrolase und einer mit dem Analyt bindefähigen Substanz.

In der heutigen Diagnostik spielt die Bestimmung von Substanzen mit Hydrolaseaktivität eine immer größere Rolle. Dies gilt sowohl für Tests zum Nachweis von Hydrolasen als auch für immunologische Tests.

Tests zum Nachweis von Hydrolasen beispielsweise in Blut sind wichtig zur Kontrolle der Körperfunktionen in der klinischen Diagnostik.

Immunologische Verfahren, in denen Hydrolasen als Markierungsenzyme verwendet werden, werden neuerdings zur Bestimmung von Analytkonzentrationen in Körperflüssigkeiten eingesetzt. Denn aus dem Vorhandensein bestimmter Komponenten lassen sich Rückschlüsse auf den Gesundheitszustand der untersuchten Personen ziehen. Störungen des Stoffwechsels bewirken oft direkt Änderungen der Konzentrationen einzelner Metabolite. Daher ist es wichtig, Verfahren zur qualitativen und quantitativen Analyse von Metaboliten zur Verfügung zu haben.

Die analytischen Methoden müssen sich aufgrund der unterschiedlichen Eigenschaften der Komponenten stark unterscheiden. Oft sind diese Testmethoden spezifisch auf eine bestimmte Komponente. Man benutzt dabei auch Eigenschaften der Komponenten, die eng mit ihrer Funktion zusammenhängen.

Ein Enzym beispielsweise kann in Körperflüssigkeiten durch die Bestimmung seiner enzymatischen Aktivität nachgewiesen werden. Die Messung der enzymatischen Aktivität erfolgt meist über Zugabe eines geeigneten Substrats. Während der Reaktion ändert sich die UV-Absorption des Reaktionsgemisches durch Bildung oder Zerstörung eines UV-absorbierenden Stoffes. Diese Absorptionsänderung kann dann in Konzentrationen bzw. Konzentrationsänderungen der zu analysierenden Komponenten umgerechnet werden.

Neuere Verfahren benutzen immunologische Schritte bei der Analyse von Komponenten in Körperflüssigkeiten. Sie finden besonders bei der Bestimmung von Haptenen, Antigenen und Antikörpern Verwendung. Immunologische Reaktionen zeichnen sich besonders durch exakte Stöchiometrie der zugrundeliegenden Reaktion zwischen den immunologischen Partnern, nämlich Hapten bzw. Antigen einerseits und Antikörpern andererseits, aus. Durch die Verwendung monoklonaler Antikörper wird außerdem die Spezifität der Nachweise stark verbessert.

Dem Fachmann sind zahlreiche Varianten von immunologischen Testverfahren bekannt. Man unterteilt sie zweckmäßig in homogene und heterogene Immuntests. Während die homogenen Verfahren darauf beruhen, daß die Nachweisreaktionen alle in Lösung stattfinden, sind bei den heterogenen Verfahren immunreaktive Verbindungen (wie Haptene, Antigene, Antikörper oder daraus gebildete Immunkomplexe) auf zwei Phasen aufgeteilt. Während ein Teil, wie beispielsweise die zu analysierende immunreaktive Komponente zu Beginn des Tests, sich in Lösung befindet, ist ein anderer Teil adsorptiv, kovalent oder präzipitativ an ein unlösliches Trägermaterial gebunden. Von den heterogenen Immuntests kennt man wiederum verschiedene Varianten, aus denen der Fachmann die geeignetste Variante je nach Art des zu bestimmenden Analyten auswählen kann. Beispielsweise wird in EP-A-0 347 839 ein immunologischer Trockentest vorgeschlagen, bei dem ein wasserunlösliches, makromolekulares Substrat, z. B. farbstoffmarkierte Stärke, eingesetzt wird. Eine Übersicht über die im Stand der Technik verwendeten Verfahren gibt Hubbuch et al. (Progress in Clinical Chemistry and Medicine, Vol. 4, Seite 109 f, Springer Verlag Berlin Heidelberg, 1986). Beispielsweise seien aufgeführt:

-    kompetitive Immuntests

Eine den Analyten in unbekannter Menge enthaltende Probe wird zusammen mit einer bekannten Menge des Analyten, der mit einer Markierung versehen ist, mit einem unlöslichen Trägermaterial in Kontakt gebracht, an das ein Immunpartner des Analyten in oben beschriebener Weise in bekannter Menge im Unterschuß gebunden ist. Durch die Immunreaktion wird ein Teil des Analyten und auch des markierten Analyten so fest gebunden, daß er bei der anschließenden Trennung der flüssigen von der festen Phase an der festen Phase verbleibt. Die Menge der an der festen bzw. in der flüssigen Phase über eine detektierbare Indikatorreaktion gemessenen Markierung ist eine Funktion der Konzentration des zu bestimmenden Analyten, welche aus der Verteilung der Markierung auf die beiden Phasen bestimmt werden kann.

Ein Beispiel hierfür ist in der EP-B-0 075 379 beschrieben.

-    unkompetitive Immuntests

a) Die Probe mit einem Analyten in unbekannter menge wird zusammen mit einem Überschuß eines zugehörigen markierten Immunpartners mit einem noch größeren Überschuß an Analyt oder Analytanalogen, welcher an eine feste Phase gebunden ist, in Kontakt gebracht. Der Überschuß des Immunpartners, der nicht in der einleitenden Immunreaktion mit dem Analyten reagiert hatte, wird so an die feste Phase gebunden. Nach der Abtrennung der festen Phase findet man in der flüssigen Phase eine zur Anfangskonzentration des Analyten proportionale Menge an Markierung.

Ein Beispiel für einen solchen Immuntest ist in der US-Patentschrift No. 4,446,232 beschrieben.

b) In einem weiteren Immuntest wird die Probe mit dem Analyten mit einem Überschuß eines zugehörigen Immunpartners, der in oben beschriebener Weise an die feste Phase gebunden ist, in Kontakt gebracht. Dann versetzt man mit einem Überschuß eines markierten Immunpartners, der eine Spezifität für einen anderen Teil des Analyten aufweist und wäscht nach der Immunreaktion den Überschuß des markierten Immunpartners von der festen Phase ab. Die an der festen Phase gemessene Markierung ist proportional zu der Menge an Analyten in der Probe. Ein solches Beispiel ist im US-Patent No. 4,098,876 beschrieben.

Als Analyt in diesen immunologischen Verfahren zur Bestimmung eines Analyten können Haptene, Antigene oder Antikörper fungieren und als Immunpartner wirken dann Antikörper, Haptene und Antigene.

Alle diese beispielhaft genannten immunologischen Verfahren beruhen auf der Bestimmung der Konzentration einer enzymmarkierten oder radioaktiv markierten immunologisch aktiven Verbindung, die in einer festgelegten Beziehung zu der zu bestimmenden Analytkonzentration steht.

Zur Bestimmung der Menge an Enzymmarkierung, wie sie beispielsweise in EP-A-0 244 932 oder in WO-A-9000 252 vorgeschlagen werden, wird der die charakteristische Menge an Indikatorenzym enthaltende Lösung ein passendes Indikatorenzymsubstrat zugegeben. Dieses wird so ausgewählt, daß eine Änderung seiner Konzentration detektierbar ist. Deshalb sind hierfür Substrate besonders geeignet, die eine charakteristische Lichtabsorption, Lichtemission oder Fluoreszenz aufweisen, oder Substrate, deren Produkte aus der Indikatorreaktion eine charakteristische Lichtabsorption, Lichtemission oder Fluoreszenz aufweisen. Als Indikatorenzym werden gelegentlich auch Hydrolasen eingesetzt. Als Substrat dienen, wie beim Nachweis von Hydrolasen selbst, Verbindungen, die durch die Hydrolase unter Anlagerung von Wasser gespalten werden, wobei ein detektierbares Produkt entsteht. Ein Beispiel für ein Substrat für die Bestimmung der Menge an Markierung mit β-Galactosidase als Indikatorenzym ist Chlorphenolrot-β-D-galactosid der EP-A-0 146 866.

Bei geringen Analytkonzentrationen sind die zu bestimmenden Mengen an Markierung auch sehr gering. Daher ist auch die in der Probe vorhandene Indikatorenzmyaktivität und die in der Zeiteinheit umgesetzte Menge an Enzymsubstrat gering. Somit dauert es sehr lange, bis die Änderung in der Konzentration des Enzymsubstrates so deutlich ist, daß sie detektiert werden kann. Durch äußere Einflüsse (Temperaturschwankungen oder ähnliches) während der langen Versuchsdauer kann der Fehler in der Messung groß werden.

Die Schwierigkeiten, die bei kleinen Analytkonzentrationen auftreten, können durch Anwendung bestimmter Verfahren vermindert werden. Beispielsweise kann die Intensität des Signals, welches durch die durch die Enzymmarkierung bewirkte Änderung in der Konzentration des Substrats bzw. Produkts der Indikatorreaktion hervorgerufen wird, erhöht werden, wenn bei der Detektion der elektromagnetischen Strahlung in Anwendung des Lambert-Beerschen Gesetzes die Meßstrecke vergrößert wird. Dies hat jedoch zur Folge, daß erheblich größere Probenvolumina benötigt werden.

Dies bedingt zum einen, daß ein Gerät zur Ausführung eines solchen Verfahrens mit großen Probenvolumina groß und wenig handlich ist.

Darüber hinaus ist es oft nicht möglich oder erwünscht, größere Probenvolumina zu verwenden. Das gilt ganz besonders bei der direkten Bestimmung von Analyten in Körperflüssigkeiten, bei denen Verfahren, die mit einem Tropfen Flüssigkeit auskommen, besonders vorteilhaft anwendbar sind. In sogenannten Trokkentests, beispielsweise der DE-A-32 476 08 sind Verfahren mit großen Probenvolumina daher nicht einsetzbar.

Wählt man ein immunologisches Analyseverfahren, bei dem die Menge an Markierung umgekehrt proportional zu der Analytkonzentration ist, so ergibt sich für sehr kleine Analytkonzentrationen ein verhältnismäßig großer Fehler, so daß ein solches Verfahren in diesem Fall schlecht anwendbar ist.

Mit Hilfe der oben beschriebenen Testverfahren können Substanzen mit Hydrolaseaktivität und somit auch Analyten in Körperflüssigkeiten je nach Testverfahren nur bis zu einer bestimmten, minimalen Konzentration bestimmt werden. Es gibt jedoch auch Bestandteile in Körperflüssigkeiten, die in noch geringerer Konzentration vorliegen und deren Bestimmung wünschenswert wäre. Die Konzentration dieser Komponenten kann mit den konventionellen Methoden gar nicht oder nur mit sehr großer Ungenauigkeit gemessen werden.

Zur Erhöhung der Empfindlichkeit von immunologischen Analyseverfahren wird in der EP-B-0 060 123 die Anwendung eines Reaktionszyklus vorgeschlagen, welcher durch das Konjugat aus alkalischer Phosphatase und einem Antikörper in Gang gesetzt wird. Durch die Enzymmarkierung wird NADP in NAD umgewandelt; NAD wird in dem Reaktionszyklus durch Alkoholdehydrogenase zu NADH reduziert und dieses wird durch ein Tetrazoliumsalz unter Bildung eines Farbstoffs wieder zu NAD oxidiert. Dieses Verfahren hat den Nachteil, daß es nicht mit Vollblut ausgeführt werden kann, da dieses selbst schon beträchtliche Konzentrationen an Phosphatasen, Dehydrogenasen sowie NADH in wechselnden Mengen enthält. Diese verfälschen den Nachweis des Konjugats. Außerdem hat dieses Verfahren den Nachteil, daß die große Anzahl von Reagentien, wie Enzymsubstraten, Enzymen oder Cofaktoren die Verfahrensausführung komplizieren und zu Stabilitätsproblemen führen können.

Auch in der EP-A-0 027 036 wird ein Verstärkungsprinzip vorgeschlagen, welches zwei Enzymsysteme umfaßt. Dieses Prinzip hat den Nachteil, daß es insbesondere im zweiten Enzymsystem mit Enzymen arbeitet, welche in Körperflüssigkeiten weit verbreitet sind und zum Teil in wechselnden Mengen vorkommen, so daß die Ergebnisse des Verfahrens verfälscht werden. Es ist weiterhin nicht möglich, die dort geschilderte Verstärkungsreaktion in einfacher Weise zu stoppen. In der Indikatorreaktion kann dort nur ein Enzym verwendet werden.

Aufgabe der vorliegenden Erfindung war es daher, ein Testmittel zur Bestimmung der Konzentration einer Substanz mit Hydrolaseaktivität bereitzustellen, welches die oben genannten Nachteile nicht aufweist.

Überraschenderweise wurde nun gefunden, daß es möglich ist, die Empfindlichkeit von Verfahren zur Bestimmung der Konzentration von Substanzen mit Hydrolaseaktivität deutlich zu erhöhen. Ein Maß für die Steigerung der Empfindlichkeit ist der Verstärkungsfaktor. Er gibt an, wieviel Mol detektierbare Einheiten (z.B. Farbstoffmoleküle) pro Mol Analyt freigesetzt werden.

Gegenstand der Erfindung sind Mittel zum Nachweis einer Substanz mit Hydrolaseaktivität in einer Probe, die ein an ein unlösliches Trägermaterial gebundenes Indikatorenzym, enthalten, das durch die Hydrolaseaktivität löslich gemacht werden kann.

Unter Substanzen mit Hydrolaseaktivität werden sowohl Hydrolasen selbst, als auch lösliche Verbindungen von Hydrolasen mit beliebigen anderen chemischen Stoffen verstanden.

Hydrolasen sind natürlich vorkommende oder künstlich hergestellte Enzyme der Hauptklasse 3. Sie sind in der Lage, charakteristische Substrate unter Verbrauch von Wasser zu spalten. Dazu gehören beispielsweise Esterasen, Peptidasen und Glycosidasen.

Besonders geeignet sind dabei Konjugate mit Endo-enzymen, wie Endoglykosidasen, Endopeptidasen usw. (Als Endoenzyme werden Enzyme bezeichnet, die nicht vom Ende des Substrats, für das sie spezifisch sind, her spalten). Als ganz besonders wirksam haben sich Konjugate aus kohlenhydratspaltenden Enzymen, wie Dextranase, Alginase, Agarase, Pectinase, Cellulase oder Chitinase herausgestellt.

Verbindungen von Hydrolasen mit anderen chemischen Stoffen sind besonders Verbindungen von Hydrolasen mit immunologisch aktiven Substanzen, wie Antigenen, Haptenen oder Antikörpern oder Immunkomplexen, aber auch Nukleinsäuren, wie DNA. Diese werden im folgenden auch als hydrolasemarkierte Verbindungen oder Hydrolasekonjugate bezeichnet.

Der erste Schritt bei einem Verfahren zum Nachweis einer Substanz mit Hydrolaseaktivität in einer Probe unter Verwendung des erfindungsgemäßen Testmittels ist das Inkontaktbringen einer Probe, welche die Substanz mit Hydrolaseaktivität enthält, mit einem an ein unlösliches Trägermaterial gebundenen Indikatorenzyms.

Unter einer Probe, in der die Substanz mit Hydrolase-Aktivität nachgewiesen werden kann, wird bevorzugt eine wässrige Lösung verstanden. Diese Lösungen können beispielsweise Lösungen einer Hydrolase oder Hydrolasekonjugats in Wasser sein. Oft werden diesen Lösungen Beimengungen, wie Salze, Detergentien etc., beispielsweise zur Erhöhung der Lagerstabilität, zugegeben. Auch in solchen Lösungen kann das erfindungsgemäße Testmittel angewandt werden. Die flüssige Probe kann auch eine Körperflüssigkeit oder eine daraus durch Zugabe oder Abtrennung von Bestandteilen erhaltene Flüssigkeit sein. Dazu gehören beispielsweise Blut, Blutplasma, Serum oder Urin.

Die flüssige Probe kann bevorzugt auch eine Flüssigkeit sein, wie sie im Verlauf von immunologischen Testverfahren entsteht. Dieses sind beispielsweise die in Annals of Clinical Biochemistry 16, 221 (1979) beschriebenen und deren vorteilhafte Fortentwicklungen, die dem Fachmann auf dem Gebiet von Immunoassays bekannt sind. In diesen Verfahren wird ein Konjugat aus einem Partner einer biospezifischen Reaktion, beispielsweise einem Antikörper gegen den im Test nachzuweisenden Analyten oder einen kompetitiven Partner eines Analyten, und einem Markerenzym, z.B. β-Galaktosidase eingesetzt. In der Bestimmungsreaktion wird die Konzentration des Markerenzyms in der resultierenden Flüssigkeit über die Enzymaktivität gemessen. In solchen Flüssigkeiten können Substanzen mit Hydrolase-Aktivität, insbesondere Konjugate von Hydrolasen mit oben genannten Verbindungen, mit dem erfindungsgemäßen Testmittel nachgewiesen werden. Diese Lösungen enthalten meistens Puffersubstanzen, Stabilisatoren, Netzmittel etc., die den Nachweis jedoch nicht stören.

In diesem Fall ist die Verwendung von Konjugaten aus Hydrolasen bevorzugt, welche normalerweise nicht in Körperflüssigkeiten vorkommen. Dazu gehören die oben genannten Hydrolasen, insbesondere jedoch Dextranase, Alginase, Agarase, Pectinase, Cellulase und Chitinase. Dextranase ist besonders bevorzugt.

Unter einem Indikatorenzym wird jedes Enzym verstanden, dessen Enzymaktivität durch Reaktion mit einem oder mehreren Substraten für dieses Enzym bestimmt werden kann. Insbesondere können alle bekannten Indikatorreaktionen, welche eine Enzym-Substrat-Reaktion ausnützen, verwendet werden. Dazu gehören beispielsweise solche, bei denen direkt ein detektierbares Produkt entsteht, aber auch solche, bei denen das Produkt der Reaktion erst in weiteren Reaktionsschritten zu einem detektierbaren Signal führt. Substrat in solchen Reaktionen, an die sich weitere Reaktionsschritte anschließen, kann beispielsweise wiederum ein Trägermaterial sein, an welches ein weiteres Indikatorenzym gebunden ist. Besonders zweckmäßig sind solche Reaktionen, in deren Verlauf eine Farbänderung stattfindet oder eine farbige Verbindung gebildet wird oder verschwindet. Solche Enzyme und dazugehörige Substrate sind dem Fachmann bekannt. Beispiele für Indikatorenzyme/Substrate sind β-Galactosidase/β-Galactoside, Peroxidase/Peroxide, sowie Phosphatasen/Phosphate usw. Als Indikatorenzym in einem bestimmten Test sind solche Enzyme besonders bevorzugt, die in der Probe nicht oder nur in vernachlässigbaren Mengen enthalten waren und dessen Aktivität sich von der Aktivität der Substanz mit Hydrolaseaktivität unterscheidet. Geeignet sind Indikatorenzyme mit einer anderen Substratspezifität als die Substanz mit Hydrolaseaktivität.

Weiterhin sind solche Indikatorenzyme bevorzugt, deren Substratspezifität durch die Substanz mit Hydrolaseaktivität nicht wesentlich beeinträchtigt wird. Ob dies auch tatsächlich der Fall ist, kann der Fachmann notfalls in wenigen Versuchen selbst ermitteln. So hat sich beispielsweise als Indikatorenzym β-Galactosidase bewährt, wenn Dextranase als Substanz mit Hydrolaseaktivität nachgewiesen werden soll.

Dieses Indikatorenzym ist vor der Zugabe der Probe, welche die Substanz mit Hydrolaseaktivität enthält, an ein in der Probe unlösliches Trägermaterial gebunden und dadurch immobilisiert, so daß es selbst unlöslich gemacht ist.

Das Trägermaterial bzw. die Art der Bindung des Indikatorenzyms an das Trägermaterial wird so gewählt, daß durch die Einwirkung der Substanz mit Hydrolaseaktivität das Indikatorenzym freigesetzt und löslich gemacht wird. Besonders bevorzugt sind folgende Fälle:

- Das Trägermaterial ist so gewählt, daß es von der Substanz mit Hydrolaseaktivität ganz oder teilweise hydrolysiert werden kann. In diesem Fall wird als Trägermaterial ein Substrat der Hydrolase in der Substanz mit Hydrolaseaktivität verwendet. Als Folge des Abbaus des Trägermaterials wird das daran ehemals gebundene Indikatorenzym freigesetzt und löst sich in der Reaktionsflüssigkeit.

Als Trägermaterial haben sich langkettige Polysaccharide oder Polysaccharidanaloge insbesondere Dextrane, Dextransulfate, Alginsäuren, Agarose, Pectine, Pectinsäuren Chitin und Carboxymethylcellulosen bewährt. Einsetzbar wären im Fall von Endopeptidasen längerkettige Peptide. Es muß in diesem Fall darauf geachtet werden, daß die Endopeptidase das Indikatorenzym selbst nicht angreift.

Dextrane sind die herkömmlich als Dextrane bezeichneten Polysaccharide sowie modifizierte und quervernetzte Dextrane, wie beispielsweise Sephadex[R]. Modifizierte Dextrane sind beispielsweise solche, die zusätzlich eingeführte funktionelle Gruppen, wie Amino- oder Carboxylgruppen aufweisen. Man kann sie herstellen, indem man auf bekannte Weise das Dextran mit Kaliumperjodat teiloxidiert und mit 1,n-Diaminoalkanen, insbesondere 1,6-Diaminohexan, bivalente Säureamide, insbesondere Succindiamid oder 1,n-Aminoalkansäuren, insbesondere 6-Aminohexansäure, umsetzt und anschließend reduziert, beispielsweise mit Natriumborhydrid.

Dadurch ist es möglich, auch eine zusätzliche Quervernetzung in den Dextranen einzuführen, beispielsweise um sie noch unlöslicher zu machen. Bevorzugt weisen die Dextrane bzw. Dextransulfate ein Molekulargewicht von ca. 40000D bis 500000D, besonders bevorzugt von 110000 bis 250000 D auf, nach einer Vernetzung $20 \times 10^6$D bis $500 \times 10^6$D.

Als Substanz mit Hydrolaseaktivität sind daher dextran-, pectin-bzw. cellulosespaltende Enzyme, wie beispielsweise Dextranase, Pectinase, Alginase bzw. Agarase besonders geeignet.

- Ebenfalls geeignete Trägermaterialien sind Verbindungen der oben genannten Materialien mit anderen anorganischen oder organischen Trägermaterialien, wie beispielsweise Polyamiden, Polyestern oder silanisiertem Titandioxid. Werden Verbindungen mit Dextranen als Trägermaterial benutzt, so können die oben beschriebenen Dextrane verwendet werden, wobei ein Molekulargewicht von $100 \times 10^6$D bis $500 \times 10^6$D besonders vorteilhaft ist. Die Substanzen mit Hydrolaseaktivität sind dann auch die oben genannten.

Diese Trägermaterialien sind entweder käuflich oder auf dem Fachmann bekannte Weise erhältlich.

So können Trägermaterialien, die Polyester enthalten, hergestellt werden, indem man Estergruppen des Polyesters verseift, mit N-Hydroxysuccinimid aktiviert und dann mit den oben genannten Dextranen, Dextransulfaten usw. umsetzt.

Polyamide oder mit Triethoxysilylpropylamin silanisiertes Titandioxid können über die freien -NH$_2$-Gruppen an die Dextrane, Dextransulfate usw. gekoppelt werden.

Die Kopplung von anorganischen oder organischen Trägermaterialien an Dextrane usw. kann auch geschehen, indem man das Dextran zunächst mit Kaliumperjodat teiloxidiert und anschließend mit dem anorganischen oder organischen Trägermaterial umsetzt.

An die so erhaltenen Dextran, Dextransulfat usw. enthaltenden Trägermaterialien ebenso wie an die nur aus Dextran, Dextransulfat usw. bestehenden Trägermaterialien kann das Indikatorenzym beispielsweise auf folgende Weise gekoppelt werden.

- Die Trägermaterialien werden mit Kaliumperjodat aktiviert und mit dem Indikatorenzym umgesetzt.

- Die Trägermaterialien werden mit einem Kupplungsreagenz und dem Indikatorenzym umgesetzt.

- Die Trägermaterialien werden mit Benzochinon oder Derivaten davon, z.B. Chloramid, und anschließend mit dem Indikatorenzym umgesetzt.

- Freie Aminogruppen der modifizierten Dextrane werden mit 3-Maleimidobenzoyl-N-succinimid (MBS) aktiviert und mit dem Indikatorenzym, insbesondere über dessen Sulfhydrylreste, gekoppelt.

Das unlösliche Trägermaterial, an welches das Indikatorenzym gekoppelt ist, kann jede beliebige Form haben, beispielsweise in Form eines Gewebes, Vlieses, Pulvers, Partikels oder Gefäßes vorliegen.

Die Menge an pro Zeiteinheit in der Reaktionsflüssigkeit gelöstem Indikatorenzym ist proportional zu der Menge an Substanz mit Hydrolaseaktivität in der Reaktionsflüssigkeit und somit im Falle eines Immuntests auch für die Konzentration des Analyten in der Probe. Voraussetzung ist, daß soviel Indikatorenzym an das Trägermaterial gebunden wurde, daß genügend abspaltbares Indikatorenzym vorhanden ist. Ein Fachmann kann die erforderliche Menge an Trägermaterial mit Indikatorenzym in wenigen einfachen Versuchen ermitteln.

Das in der Reaktionsflüssigkeit gelöste Indikatorenzym wird anschließend von dem an das unlösliche Trägermaterial gebundenen Indikatorenzym abgetrennt. Dies geschieht in besonders einfacher Weise durch Entfernen der Reaktionsflüssigkeit, beispielsweise durch Absaugen, Abpipettieren, Abfiltrieren, Dekantieren, Abzentrifugieren usw.

Dadurch kann durch in der Reaktionsflüssigkeit gegebenenfalls gelöste Substanz mit Hydrolaseaktivität ab diesem Zeitpunkt kein weiteres Indikatorenzym mehr in der abgetrennten Reaktionsflüssigkeit löslich gemacht werden. Dies ist von Vorteil, da die Reaktion so auf besonders einfache Weise zu einem genau festgelegten Zeitpunkt gestoppt wird.

Zur Bestimmung der Menge an freiem Indikatorenzym in der vom Trägermaterial getrennten Lösung wird diese unter Bedingungen, die für jedes Indikatorenzym konstant und dem Fachmann bekannt sind, zu einem bestimmten Zeitpunkt einem geeigneten Substrat zugesetzt. Zu einem bestimmten späteren Zeitpunkt wird die Menge an verbrauchtem Substrat oder gebildetem Produkt gemessen. Ein Beispiel für eine solche detektierbare Indikatorreaktion ist für β-Galactosidase in EP-A-0 146 866 beschrieben.

Der Verlauf der Reaktion kann bei Verwendung von chromogenen Substraten durch visuelle Beobachtung verfolgt werden. Quantitative Auswertungen werden jedoch zweckmäßigerweise mit Hilfe von Apparaten, beispielsweise Photometern auch Fluoreszenzphotometer oder Reflexionsphotometern vorgenommen.

Die Konzentration an freiem Indikatorenzym ist ein genau festgelegtes Maß für die Menge der Substanz mit Hydrolaseaktivität.

Zur Korrelierung des Ergebnisses der Indikatorreaktion mit einer bestimmten Konzentration an Substanz mit Hydrolaseaktivität wird bevorzugt eine Eichkurve mit Proben mit unterschiedlichen, aber bekannten Konzentrationen bei sonst gleichen Bedingungen aufgenommen.

Nach Ermittlung des Ergebnisses der Indikatorreaktion unter denselben Bedingungen mit einer Probe mit unbekannter Konzentration kann die gesuchte Konzentration aus der Eichkurve direkt abgelesen werden.

Eine wichtige Bedingung für korrekte Ergebnisse ist, daß das in der Indikatorreaktion eingesetzte Substrat möglichst nicht mit noch an Trägermaterial gebundenem Indikatorenzym in Berührung kommt. Dies wird im allgemeinen durch die erfindungsgemäße räumliche Trennung von freiem und gebundenem Indikatorenzym erreicht. Darüber hinaus wird die Sicherheit des Ergebnisses noch erhöht, wenn das Substrat, dem die Lösung mit dem Indikatorenzym zugesetzt wird, an eine feste Matrix gebunden vorliegt. Diese Matrix kann ein Reagenzfilm oder Gewebe, Papier oder Partikel sein.

Die Bindung des Substrats erfolgt so, daß die Umsetzung mit dem Indikatorenzym nicht wesentlich beeinträchtigt wird. Dies kann beispielsweise durch Verknüpfung des Substrats über Abstandshalter (Spacer) erreicht werden.

Das Substrat und die zur Ausführung benötigten Reagenzien können jedoch auch in Lösung, in trockener Form, in Form eines Überzugs auf eine Matrix oder im Gemisch mit Substanzen, die der Mischung insgesamt einen festen Aggregatzustand verleihen und die Mischung so leicht handhabbar und dosierbar machen (Strukturbildner) vorliegen.

Das Verfahren wird unter den Bedingungen durchgeführt, wie sie für den Ablauf der beteiligten Reaktionen, insbesondere der Enzymreaktionen, optimal sind.

Die Temperatur wird so gehalten, daß keine Inaktivierung der Enzyme stattfindet, daß aber die Reaktionsgeschwindigkeit noch ausreichend hoch ist. Der bevorzugte Temperaturbereich liegt daher bei 18°C bis 42°C, besonders bevorzugt bei 25°C bis 37°C.

Der pH-Wert der einzelnen Reaktionsschritte kann gleich oder verschieden sein. Da die beteiligten Enzyme ein pH-Optimum aufweisen, ist es vorteilhaft, in dessen Nähe zu arbeiten. Dextranase hat z. B. ein pH-Optimum von 6.0, Pectinase von 4.0, Cellulase von 5.0 und β-Galactosidase von 7.4. Als pH-Bereich, bei dem noch ausreichend schnelle Reaktion stattfindet, kann daher pH 3.0 - 9.0, insbesondere 3.5 - 8.0 benutzt werden. Durch Umpuffern kann aber auch für jeden Reaktionsschritt der optimale pH eingestellt werden.

Mit dem erfindungsgemäßen Testmittel ist ein sehr empfindliches Bestimmungsverfahren möglich, mit dem man auch sehr schnelle Konzentrationsbestimmungen durchführen kann. Das Verfahren ist umso schneller, je größer der Verstärkungsfaktor ist. Im allgemeinen ist es ohne weiteres möglich, eine Bestimmung innerhalb von weniger als 1 h, bevorzugt weniger als 10 min, durchzuführen.

Mit dem Testmittel ist das Bestimmungsverfahren so empfindlich, daß unter normalen Bedingungen sogar Konzentrationen von bis zu $10^{-12}$ mol/l an Substanz mit Hydrolaseaktivität innerhalb von 10 min nachgewiesen werden können. Es können aber auch größere Konzentrationen gemessen werden; dann kann das Ergebnis der Bestimmung schon in noch kürzerer Zeit erhalten werden. Dies ist für die klinische Diagnostik von großem Vorteil.

Weitere Vorteile beim Einsatz des erfindungsgemäßen Testmittels sind, daß Bestimmungsverfahren sehr einfach durchzuführen sind, nur sehr wenige Komponenten umfassen müssen, nämlich das an das Trägermaterial gebundene Indikatorenzym und ein Indikatorenzymsubstrat, welches in der Indikatorreaktion zu einem meßbaren Signal führt, daher wenig Stabilitätsprobleme und Probleme mit Nebenreaktionen mit sich bringt und das mit dem Testmittel durchgeführte Verfahren durch Wahl der Indikatorreaktion so angepaßt werden kann, daß auch andere Komponenten in beispielsweise Körperflüssigkeiten die Reaktion nicht unberechenbar beeinflussen.

Das erfindungsgemäße Testmittel wird besonders bevorzugt in Form sogenannter Teststreifen ausgeführt. Sie bestehen hauptsächlich aus einer Grundplatte oder Folie, auf welcher die für den Test erforderlichen Reagenzien meist mittels Vliesen oder Filmen angebracht sind. Dann ist das Trägermaterial, an welches das Indikatorenzym gebunden ist, in vorteilhafter Weise als ein Vlies ausgebildet, auf welches die zu untersuchende Probe aufgegeben wird. In einem weiteren Vlies oder Film befindet sich die zur Indikatorreaktion benötigten Reagenzien. In diesem Vlies kann sofort die Auswertung des Tests beispielsweise visuell oder photometrisch erfolgen.

Besondere Verwendung kann das erfindungsgemäße Testmittel zum Nachweis einer Substanz mit Hydrolaseaktivität beispielsweise in immunologischen Verfahren zum Nachweis eines Analyten finden. Solche Verfahren sind dem Fachmann auf dem Gebiet der Immunoassays bekannt (beispielsweise Annals of Clinical Biochemistry 16, 221 (1979)). Diese Verfahren werden dahingehend abgewandelt, daß anstelle eines Indikatorenzymkonjugats ein Hydrolasekonjugat verwendet wird und der darin erforderliche Nachweis des Konjugats unter Verwendung des erfindungsgemäßen Testmittels durchgeführt wird.

Verfahren, welche die Synthese von enzymmarkierten und auch hydrolasemarkierten Immunpartnern, wie Haptenen, Antigenen, Antikörpern und Antikörperfragmenten betreffen, sind dem Fachmann bekannt oder sie können analog zu bekannten Verfahren durchgeführt werden.

Unter Analyten werden sowohl Haptene und Antigene als auch Antikörper verstanden. Haptene sind Substanzen mit relativ niedrigem Molekulargewicht, die zwar von Antikörpern erkannt werden, jedoch allein keine Immunantwort auslösen können. Beispiele für solche Haptene sind endogene Substanzen wie Thyroxin (T4), Trijodthyronin (T3), oder therapeutisch wirksame Verbindungen wie Digoxin, Theophyllin oder Drogen (Rauschgifte).

Unter Antigenen werden Proteine verstanden, die von Antikörpern erkannt werden und eine Immunantwort auslösen können. Diese Proteine können natürlich vorkommende oder künstlich hergestellt sein. Zu den Antigenen zählen weiterhin Antikörper, die von einem anderen Antikörper als Antigen erkannt werden können. Beispiele von Antigenen, deren Bestimmung in Körperflüssigkeiten wichtig ist, sind Thyreotropin (TSH), follikelstimulierendes Hormon (FSH), luteinisierendes Hormon (LH), Hämoglobin (Hb), humanes Choriogonadotropin (hCG), Immunglobulin G, humanes Serum Albumin (hSA), Carcinoembryonales Antigen (CEA), alpha-Foetoprotein (AFP) und auch Enzyme. Außerdem zählen zu den Antigenen die natürlichen Derivate von Proteinen, wie beispielsweise Glykoproteine, oder künstlich hergestellte Verbindungen von Proteinen mit anderen chemischen Verbindungen.

Unter Antikörpern werden Immunglobuline verstanden, die zur Bindung eines Antigens oder Haptens der obigen Definition in der Lage sind.

Für den Fall, daß der Analyt ein Antigen oder Hapten ist, haben sich folgende Verfahren besonders bewährt:

- Die den Analyten enthaltende Probenlösung wird mit einem Überschuß an Konjugat aus einem Antikörper gegen den Analyten (analytspezifische Substanz) und einer Hydrolase versetzt. Es bildet sich ein Immunkomplex aus Analyt und Konjugat. Der Überschuß an Konjugat wird durch Immunreaktion an einen Träger gebunden, an den ein Überschuß an Analyt oder einer analytanalogen Verbindung gebunden ist. Eine analytanaloge Verbindung ist eine Verbindung, welche sich in ihren Wechselwirkungen mit Reaktionspartnern, z.B. in immunologischen Reaktionen ähnlich verhält wie der Analyt. Im allgemeinen hat ein Analytanaloges eine nur wenig vom Analyten unterschiedene Struktur. Eine analytanaloge Verbindung kann auch einen antiidiotypen Antikörper bedeuten.

Antiidiotypen-Antikörper sind gegen die Antigenbindungsstelle eines Antikörpers gerichtet und reagieren mit dieser Bindungsstelle wie der Analyt. Die das Konjugat aus Hydrolase und Immunkomplex enthaltende Lösung wird vom Träger getrennt und gemäß dem erfindungsgemäßen Verfahren behandelt. Man erhält ein Versuchsergebnis für das Hydrolasekonjugat, aus welchem man auf die Anwesenheit bzw. auf die Menge des Analyten schließen kann. Diese Ausführungsform ist besonders bevorzugt, weil keine exakte Dosierung des Konjugats und des Trägermaterials erforderlich ist. Als Antikörper wird bevorzugt ein monoklonaler Antikörper verwendet. Besonders bevorzugt sind Fragmente, beispielsweise Fab. Die Stöchiometrie Fab:

Enzym liegt bevorzugt bei 1:2-1:4.

- Die den Analyten enthaltende Probenlösung wird mit einem Überschuß an Konjugat aus einem Antikörper gegen den Analyten (analytspezifische Substanz) und einer Hydrolase über die Menge an voraussichtlich vorhandenen Analyten versetzt. Die Menge an Konjugat ist hierbei bekannt. Es bildet sich ein Immunkomplex aus Analyt und Konjugat. Der verbleibende Überschuß an Konjugat wird durch Immunreaktion an einen Träger gebunden, an den ein Überschuß eines Antikörpers gegen den Analyten (analytspezifische Substanz), der eine andere Bindungsstelle des Analyten erkennt als der Antikörper des Konjugats, gebunden ist. Die den Überschuß des Konjugats aus Hydrolase und Antikörper enthaltende Lösung wird vom Träger getrennt und nach dem erfindungsgemäßen Verfahren das in der Lösung enthaltene Konjugat aus Antikörper und Hydrolase nachgewiesen.

- Die den Analyten enthaltende Probenlösung wird mit einer bekannten Menge an Konjugat aus Analyt oder Analytanalogon und einer Hydrolase versetzt. Das Gemisch wird auf einen Träger aufgegeben, an welchen ein bekannter Unterschuß eines Antikörpers gegen den Analyten (analytspezifische Substanz) und das Analytanalogon bezogen auf die Summe aus Analyt und Konjugat gebunden ist.

  Ein Teil des Analyten und des Konjugats wird an den Träger gebunden. Nach dem erfindungsgemäßen Verfahren kann nach Abtrennung der Lösung das in der Lösung befindliche Konjugat nachgewiesen werden.

Für den Fall, daß der Analyt ein Antikörper ist, können die gleichen Prinzipien angewandt werden, jedoch muß dann anstelle der Antikörper in den oben beschriebenen Verfahren ein Antigen oder ein gegen den Antikörper gerichteter Antikörper verwendet werden.

In immunologischen Verfahren zum Nachweis eines Analyten schließt sich an den Nachweis des Hydrolasekonjugats unter Verwendung des erfindungsgemäßen Testmittels die Auswertung an. Aus der Anwesenheit oder bei quantitativer Auswertung der Menge des nachgewiesenen Hydrolasekonjugats kann nämlich auf die Anwesenheit bzw. die Menge des Analyten in der ursprünglich eingesetzten Probe geschlossen werden. Dies kann beispielsweise über eine Eichkurve geschehen.

Die Vorteile des erfindungsgemäßen Testmittels zum Nachweis einer Substanz mit Hydrolaseaktivität wirken sich auch in diesem immunologischen Verfahren zum Nachweis eines Analyten aus. Insbesondere ist es von Vorteil, daß die Substanz mit Hydrolaseaktivität eine Hydrolaseaktivität aufweist, die in den zu untersuchenden Lösungen nicht enthalten ist. Das erfindungsgemäße Testmittel ist auch für Analyten geeignet, die in nur sehr geringen Mengen in Proben, z.B. Körperflüssigkeiten vorhanden sind, da es einen Verstärkungseffekt aufweist, der mit einfachen Mitteln erreicht wird.

In analoger Weise kann das erfindungsgemäße Testmittel auch beim Nachweis von Nukleinsäuren angewendet werden, welche sich enzymmarkierter Nukleinsäuren bedienen, beispielsweise bei Nukleinsäurehybridisierungstests. Als Enzymmarkierung werden dann Hydrolasen verwendet, welche dann mit dem erfindungsgemäßen Testmittel nachgewiesen werden. Unter Nukleinsäuren wird insbesondere DNS verstanden.

Das erfindungsgemäße Testmittel enthält alle zur Durchführung der Bestimmung der Konzentration der Substanz mit Hydrolaseaktivität benötigten Reagenzien.

Insbesondere enthält es ein an ein unlösliches Trägermaterial gebundenes Indikatorenzym, welches durch die Hydrolaseaktivität löslich gemacht werden kann.

Außerdem enthält es zweckmäßigerweise die Reagenzien, die zur Bestimmung der Konzentration des Indikatorenzyms erforderlich sind. Diese Reagenzien enthalten beispielsweise ein Indikatorenzymsubstrat.

Indikatorenzymsubstrate sind Verbindungen, welche durch Katalyse durch das Indikatorenzym eine nachweisbare Änderung vollziehen. Sie sind bevorzugt spaltbar oder Bestandteile eines Redoxsystems. Der Nachweis des Substrats beziehungsweise des Produkts seiner Reaktion mit dem Indikatorenzym kann beispielsweise colorimetrisch, fluorimetrisch oder auch elektrochemisch vorgenommen werden. Beispiele solcher Substrate sind für $\beta$-Galaktosidase die Galaktoside von Resorufin, Chlorphenolat oder Nitrophenol. Beispiele für Peroxidasesubstrate sind Resorufin und Triarylimidazole.

Außerdem können die Reagenzien erforderlichenfalls pH-Puffersubstanzen, Stabilisatoren, Aktivatoren etc. enthalten.

Die Art des Indikatorenzymsubstrats sowie die weiteren Inhaltsstoffe richten sich nach dem zu bestimmenden Indikatorenzym und sind dem Fachmann bekannt. Es wird beispielsweise $\beta$-Galactosidase als Indikatorenzym verwendet, so können die in der EP-A-0 146 866 aufgeführten Reagenzien benutzt werden.

Die Reagenzien, die zur Bestimmung der Konzentration des Indikatorenzyms verwendet werden, werden bevorzugt vor und während des Bestimmungsverfahrens von dem an das Trägermaterial gebundenen Indikatorenzym getrennt gehalten, um keine vorzeitige, die Bestimmung verfälschende Indikatorreaktion zu erhalten.

Das erfindungsgemäße Mittel kann das an das unlösliche Trägermaterial gebundene Indikatorenzym als Suspension, Pulver, Vlies, Gewebe, Gefäß, Tablette usw. gegebenenfalls zusammen mit bekannten Trägersubstanzen enthalten.

Die für die Indikatorreaktion erforderlichen Reagenzien des erfindungsgemäßen Mittels können in Form eines Pulvers, einer Tablette, einer Lösung oder auf ein Vlies, Gewebe usw. imprägniert vorliegen.

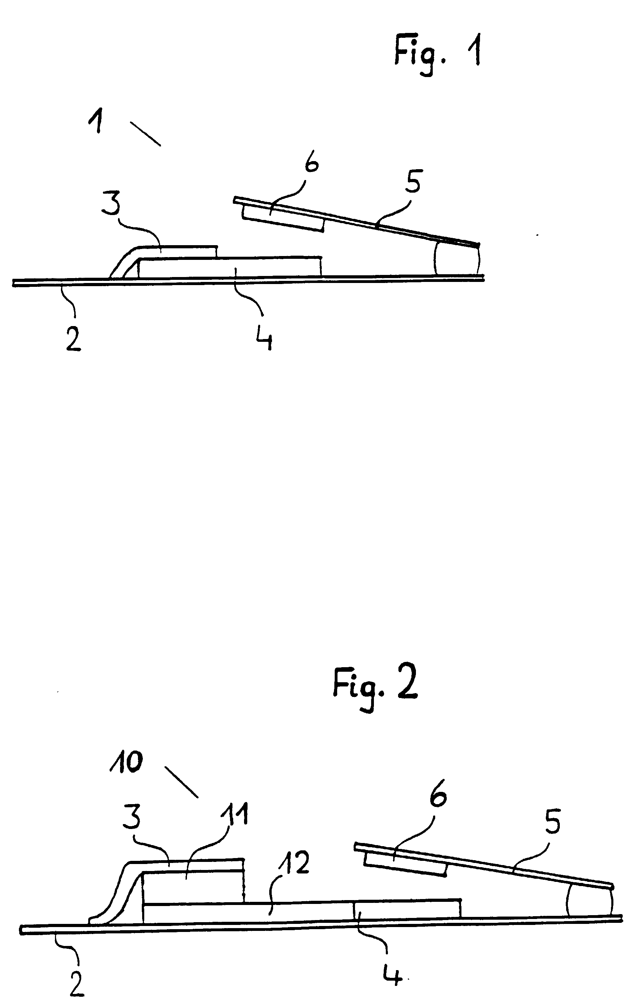

Eine bevorzugte Ausführungsform 1 des Mittels in Form eines Teststreifens zeigt Fig. 1. Der Teststreifen weist mehrere von der Probe durchströmbare Schichten auf, die entweder zur Abtrennung von Substanzen in der Probe, zur Inkubation mit Reagenzien, zum Transport oder zum Beobachten von Reaktionen dienen. Bevorzugt stehen diese Schichten so miteinander in Kontakt, daß die Probe durch Kapillarkräfte von einer Schicht zur anderen gelangt. Vlies 3 dient zur Abtrennung von störenden Bestandteilen der Probe, wie Erythrozyten bei der Untersuchung einer Blutprobe (DE-A-3029579). Hier wird die Probe aufgegeben. 4 ist das Trägermaterial, an welches das Indikatorenzym gebunden ist, bei der Bestimmung von Substanzen mit Dextranaseaktivität ist dies beispielsweise ein Dextran, an das ein Indikatorenzym gebunden ist. Die hierher durch Vlies 3 gelangte Probe verteilt sich in dieser Zone, wobei durch die Einwirkung der Substanz mit Hydrolaseaktivität ein Teil des Indikatorenzyms vom Trägermaterial abgespalten wird und in Lösung geht. Nach Umlegen der Klappe 5 mit einem Substratvlies 6 auf das auf der Grundfolie 2 befindliche Trägermaterial 4 dringt die Reaktionslösung in das Substratvlies 6 ein und die Indikatorreaktion wird gestartet.

Ein weiterer Gegenstand der Erfindung ist ein Testmittel zum Nachweis oder Bestimmung eines Analyts in Körperflüssigkeiten unter Verwendung eines Konjugats aus einem Markierungsenzym und einer mit dem Analyten bindefähigen Substanz, wobei als Markierungsenzym eine normalerweise nicht in der Körperflüssigkeit vorkommende Hydrolase verwendet wird, und eine für die Analytmenge charakteristische Menge dieses Hydrolasekonjugats mit einem an ein unlösliches Trägermaterial gebundenen Indikatorenzym in Kontakt gebracht wird, wobei durch Einwirkung der Hydrolase das Indikatorenzym ebenfalls in Abhängigkeit der Menge an Analyt abgespalten wird, das abgespaltene Indikatorenzym von dem Trägermaterial abgetrennt und die Aktivität dieses Indikatorenzyms bestimmt wird.

Das erfindungsgemäße Mittel zur Bestimmung der Konzentration eines Analyten enthält alle Bestandteile des oben geschilderten Mittels zur Bestimmung der Konzentration einer Substanz mit Hydrolaseaktivität. Zusätzlich enthält es Reagenzien, welche es erlauben, eine für die zu bestimmende Analytkonzentration charakteristische Menge an Substanz mit Hydrolaseaktivität freizusetzen. Diese Reagenzien können vom Fachmann analog zu den bekannten Verfahren eingesetzt werden.

Gegenstand der Erfindung ist ein Testmittel zum Nachweis eines Analyten in einer flüssigen Probe, welches aus mehreren von der Probe durchströmbaren Schichten aufgebaut ist, die an einem festen Träger befestigt sind, dadurch gekennzeichnet, daß es in folgender Reihenfolge enthält:

- eine Schicht, welche eine Konjugat aus einem Antikörper gegen den Analyten mit einer in der Probe nicht vorhandenen Hydrolase enthält,

- eine Schicht, welche immobilisierten Analyten oder immobilisiertes Analytanaloges enthält,

- eine Schicht, welche eine an ein unlösliches Trägermaterial gebundenes Indikatorenzym enthält, wobei das Indikatorenzym durch Inkontaktbringen mit einer Hydrolase löslich gemacht werden kann und

- eine Schicht, welche die für den Nachweis des Indikatorenzyms erforderlichen Reagenzien enthält, wobei jede Schicht mit der Nachbarschicht in Kontakt steht oder in Kontakt gebracht werden kann.

Eine Ausführungsform eines solchen Mittels ist in Fig. 2 abgebildet. Sie betrifft einen sogenannten immunenzymometrischen Assay. Teststreifen 10 enthält zusätzlich zu der oben beschriebenen Ausführungsform 1 die Vliese 11 und 12. Auf Vlies 11 ist ein Konjugat aus Immunpartner des zu bestimmenden Analyten, welcher durch eine Hydrolase markiert ist, im Überschuß zu dem Analyten imprägniert. Vlies 12 enthält eine Matrix, an welche der Analyt oder Analytanaloges im Überschuß gebunden ist.

Zur Bestimmung der Konzentration eines Analyten in einer Probe wird die Probe auf Vlies 3 aufgegeben. Nach Eindringen der Lösung in Vlies 11 wird daraus das Konjugat gelöst und reagiert mit dem in der Probe anwesenden Analyten in einer Immunreaktion. Die Lösung mit dem gebildeten Immunkomplex und dem Überschuß an Konjugat dringt in das Vlies 12 ein. Dort wird der Überschuß an Konjugat durch Immunreaktion mit dem matrixgebundenen Analyten oder Analytanalogen aus der Lösung entfernt. Die Lösung mit dem Immunkomplex, welcher die Substanz mit Hydrolaseaktivität darstellt, dringt in Vlies 4 ein und wird wie oben beschrieben bestimmt. Die Menge an Substanz mit Hydrolaseaktivität ist direkt proportional zu der Menge des Analyten in der Probe.

Die in Fig. 1 und 2 gezeigten Testmittel können außerdem, falls zur Durchführung vorteilhaft, weitere Bestandteile, insbesondere Vliese oder Gewebe, evtl. reaktiv beschichtet, enthalten. Die Anordnung der Vliese kann vom Fachmann der Testführung entsprechend gewählt werden.

Das Bestimmungsverfahren unter Verwendung des erfindungsgemäße Testmittels wird vorteilhaft in einem Analysenautomaten durchgeführt, da die Ergebnisse dann schnell zu erhalten und gut reproduzierbar sind.

Die folgenden Beispiele dienen dazu, die Erfindung näher zu erläutern.

Beispiel 1

Herstellung eines Mittels zur Bestimmung der Konzentration von Dextranase oder Dextranase-Konjugat

A) Herstellung von an unlösliches Dextran gebundener β-Galactosidase

a) Herstellung von oxidiertem Dextran

50 mg trockenes unlösliches Dextran (Charakterisierung: Sephadex®6200) werden in 10 ml bidestilliertem Wasser bei 90 °C 2h lang gequollen. Das abgesetzte Gel wird mit 2 ml Kaliumperjodatlösung (15 mmol/l in bidestilliertem Wasser) für 30 min. bei Raumtemperatur unter Schütteln umgesetzt.

Das erhaltene Gel wird fünfmal mit 10 ml 50 mM Kaliumphosphatpuffer (pH 8.5) gewaschen und jeweils in einer Glasfritte filtriert.

b) Kopplung

25 mg β-Galactosidase (Boehringer Mannheim GmbH) werden in 0.5 ml Kaliumphosphatpuffer (50 mM, pH 8.5) gelöst und 1 ml oxidiertes Dextran aus a) dazugegeben. Man koppelt bei 4 °C auf einem Rollenmischer über Nacht.

c) Reduktion der Azomethinbindungen

Nach der Kopplung wird das Gel mit 100 mM Natriumboratpuffer (pH 8.5) gewaschen und mit 1 ml Natriumborhydridlösung (5 mg/ml in 100 mM Natriumboratpuffer, pH 8.5) bei 0°C 1 h lang reduziert. Anschließend wird das Gel mit Tween-Puffer (10 mM Kaliumphosphatpuffer, pH 7.0; 25 mM Natriumchlorid; 0.05 % Tween[R] 20) gewaschen, bis keine β-Galactosidaseaktivität mehr zu messen ist.

B) Als Substratlösung dient eine 2 mM Lösung von 2-Nitrophenol-β-galactosid

Beispiel 2

Bestimmung der Konzentration von Dextranase oder Dextranase-Konjugat in einer Probenlösung

Als Probenlösung dienten eine $2 \cdot 10^{-11}$ M und eine $2 \cdot 10^{-12}$ M Lösung von Dextranase oder Dextranase-Konjugat (Dextranase = 1,6-Glucan-Glucanohydrolase: EC 3.2.1.11, Firma Miles, spezifische Aktivität 1700 U/mg) in Tween-Puffer (100 mM Kaliumphosphat, pH 6.0; 0.05 % Tween[R] 20; 5 mg/ml Crotein[R] C) .

Zur Durchführung des Tests wurden 1000 µl der in Beispiel 1A) hergestellten an unlösliches Dextran gebundenen β-Galactosidase und 100 µl der Probenlösung vermischt und 10 min bei 37 °C geschüttelt. Anschließend wurde die Suspension zentrifugiert und der Überstand mit 500 mM Kaliumphosphatpuffer auf pH 8.5 eingestellt.

0,1 ml des Überstands wurden mit 1 ml der Substratlösung versetzt und die Absorption bei 37 °C im Vergleich zu einer gleichbehandelten Referenzlösung, (d. h. ohne Dextranase-bzw. Dextranasekonjugat-Zusatz) gemessen.

Beispiel 3

Bestimmung des Verstärkungsfaktors

Der Verstärkungsfaktor ist hier definiert als das Verhältnis der durch die Einwirkung der Dextranase bzw. des Konjugats aus dem mit β-Galactosidase gekoppelten Trägermaterial aus Beispiel 1 A) freigesetzten Menge an β-Galactosidase zu der eingesetzten Menge an Dextranase.

$$V = \frac{\text{Aktivität (Units) der freigesetzten } \beta\text{-Galaktosidase}}{\text{Aktivität des eingesetzten Dextranasekonjugats}}$$

Aktivität ist der Umsatz von Substrat pro Zeiteinheit. Der Verstärkungsfaktor wurde analog zu Beispiel 2 ermittelt, wobei die Inkubationszeit variiert wurde.

| Inkubationszeit | Verstärkungsfaktor |
|---|---|
| 5 min. | 850 |
| 10 min. | 1650 |
| 15 min. | 2500 |
| 20 min. | 3300 |
| 25 min. | 4100 |

**Patentansprüche**

1. Testmittel zum Nachweis einer Substanz mit Hydrolaseaktivität in einer flüssigen Probe, welches aus mehreren von der Probe durchströmbaren Schichten aufgebaut ist, die auf einem Testträger (2) befestigt sind, dadurch gekennzeichnet, daß es eine Schicht (4), welche an ein unlösliches Trägermaterial gebundenes Indikatorenzym enthält, wobei das Indikatorenzym durch Inkontaktbringen mit einer Hydrolase löslich gemacht werden kann und eine Schicht (6), welche die für den Nachweis des Indikatorenzyms erforderlichen Enzymsubstrate und gegebenenfalls weitere Reagenzien enthält, wobei diese Schicht (6) mit der Schicht (4) mit dem Indikatorenzym in Kontakt gebracht werden kann, enthält.

2. Testmittel zum Nachweis eines Analyten in einer flüssigen Probe, welches aus mehreren von der Probe durchströmbaren Schichten aufgebaut ist, die an einem festen Träger (2) befestigt sind, dadurch gekennzeichnet, daß es in folgender Reihenfolge enthält

- eine Schicht (11), welche ein Konjugat aus einem Antikörper gegen den Analyten mit einer in der Probe nicht vorhandenen Hydrolase enthält,

- eine Schicht (12), welche immobilisierten Analyten oder immobilisiertes Analytanaloges im Überschuß enthält,

- eine Schicht (4), welche an ein unlösliches Trägermaterial gebundenes Indikatorenzym enthält, wobei das Indikatorenzym durch Inkontaktbringen mit einer Hydrolase löslich gemacht werden kann und

- eine Schicht (6), welche die für den Nachweis des Indikatorenzyms erforderlichen Enzymsubstrate und gegebenenfalls weitere Reagenzien enthält, wobei jede Schicht mit der Nachbarschicht in Kontakt steht oder in Kontakt gebracht werden kann

und dann, wenn der zu bestimmende Analyt ein Antikörper ist, in vorstehenden Schichten auch Antikörper gegen Analyt und Analyt gegen Antikörper ausgetauscht sein können.

## Claims

1. Test means for the detection of a substance having hydrolase activity in a liquid sample which is composed of several layers through which the sample can flow which are attached to a test support (2), wherein it contains a layer (4) which contains an indicator enzyme bound to an insoluble support material and the indicator enzyme can be rendered soluble by being brought into contact with a hydrolase and it contains
a layer (6) which contains the enzyme substrates required for the detection of the indicator enzyme and optionally further reagents and this layer (6) can be contacted with the layer (4) containing the indicator enzyme.

2. Test means for the detection of an analyte in a liquid sample which is composed of several layers through which the sample can flow which are attached to a solid carrier (2), wherein it contains in the following order:

- a layer (11) which contains a conjugate comprising an antibody to the analyte together with a hydrolase not present in the sample,

- a layer (12) which contains an excess of immobilized analyte or immobilized analyte analogue,

- a layer (4) which contains an indicator enzyme bound to an insoluble support material and the indicator enzyme can be rendered soluble by being brought into contact with a hydrolase and

- a layer (6) which contains the enzyme substrates required for the detection of the indicator enzyme and optionally further reagents wherein each layer is in contact or can be brought into contact with the adjacent layer

and, if the analyte to be determined is an antibody, it is then also possible in the above-mentioned layers to substitute antibody by analyte and analyte by antibody.

## Revendications

1. Moyen de test pour détecter une substance ayant une activité d'hydrolase dans un prélèvement liquide, qui est constitué de plusieurs couches pouvant être traversées par le prélèvement, qui sont fixées sur un support de test (2), caractérisé en ce qu'il contient une couche (4) laquelle contient une enzyme indicatrice liée à une matière support insoluble, dans lequel l'enzyme indicatrice peut être rendue soluble par mise en contact avec une hydrolase et ne couche (6), qui contient les substrats à enzyme nécessaires à la détection de l'enzyme indicatrice et éventuellement un complément de réactifs, dans lequel cette couche (6) avec la couche (4) peut être mise en contact avec l'enzyme indicatrice.

2. Moyen de test pour détecter un analyte dans un prélèvement liquide, qui est constitué de plusieurs couches pouvant être traversées par le prélèvement, qui sont fixées sur un support de test (2), caractérisé en ce qu'il contient dans l'ordre suivant

- une couche (11), qui contient un conjugué à base d'un anticorps contre l'analyte avec une hydrolase qui ne se trouve pas dans le prélèvement,

- une couche (12), qui contient en excès de l'analyte immobilisée ou un analogue d'analyte immobilisé,

- une couche (4), qui contient une enzyme indicatrice liée à une matière support insoluble, dans lequel l'enzyme indicatrice peut être rendue soluble par une mise en contact avec une hydrolase et

- une couche (6), qui contient les substrats à enzyme nécessaires à la détection de l'enzyme indicatrice et éventuellement un complément de réactifs, dans lequel chaque couche est en contact ou peut être mise en contact avec la couche voisine et alors, lorsque l'analyte à déterminer est un anticorps, les anticorps peuvent être replacés par l'analyte et l'analyte par les anticorps dans les couches précédentes.

Fig. 1

Fig. 2